# EUROPEAN PATENT APPLICATION

(11) **EP 2 182 382 A1**
(43) Date of publication of application: **05.05.2010**
(21) Application number: 09173573.8
(22) Date of filing: 21.10.2009
(51) Int. Cl.: G01S 7/52, G01S 15/89, G06T 17/40

(54) **Ultrasound system and method for providing three-dimensional ultrasound images**

(30) Priority: 03.11.2008 KR 20080108559; 27.05.2009 KR 20090046512
(71) Applicant: MEDISON CO., LTD., Kangwon-do 250-870 (KR)
(72) Inventor: Lee, Suk Jin, 135-851 Seoul (KR)
(74) Representative: Schmid, Wolfgang

(57) **Abstract**

The present invention relates to an ultrasound system and method capable of providing three-dimensional ultrasound images. The ultrasound system of the present invention transmits ultrasound signals to a target object, receives ultrasound echo signals reflected from the target object and acquires ultrasound data based on the ultrasound echo signals. The ultrasound system allows a user to input rendering setting information containing information on at least two rendering directions. The ultrasound system forms volume data by using the ultrasound data, renders the volume data along the at least two rendering directions and forms three-dimensional ultrasound images corresponding to the at least two rendering directions. The ultrasound system stores the three-dimensional ultrasound images. The ultrasound system displays the three-dimensional ultrasound images on a display region.

## Description

The present application claims priority from Korean Patent Application Nos. 10-2008-0108559 (filed on November 3, 2008) and 10-2009-0046512 (filed on May 27, 2009) the entire subject matters of which are incorporated herein by reference.

### BACKGROUND OF THE INVENTION

### [Technical Field]

The present invention generally relates to ultrasound systems, and more particularly to an ultrasound system and method for providing at least two three-dimensional ultrasound images by performing rendering upon volume data in at least two directions based on a user's desired rendering directions.

### [Background Art]

The ultrasound system has become an important and popular diagnostic tool due to its non-invasive and non-destructive nature. Modem high-performance ultrasound imaging diagnostic systems and techniques are commonly used to produce two or three-dimensional images of internal features of patients.

An ultrasound system generally uses a probe containing an array of piezoelectric elements to transmit and receive ultrasound signals. The ultrasound system forms a volume data based on the received ultrasound signals and further forms three-dimensional ultrasound images by rendering the volume data. The ultrasound system displays the three-dimensional ultrasound images on a monitor or screen to observe the same.

Generally, the ultrasound system may render the volume data along a predetermined direction to form the three-dimensional ultrasound image. Thus the, the ultrasound system may not provide the three-dimensional ultrasound images of desirable viewing directions based on the user's request.

### BRIEF DESCRIPTION OF THE DRAWINGS

- FIG. 1: is a block diagram showing an illustrative embodiment of an ultrasound system.
- FIG. 2: is a block diagram showing a configuration of an ultrasound data acquisition unit located within the ultrasound system.
- FIG. 3: is a schematic diagram showing a memory within the ultrasound system.
- FIG. 4: is a schematic diagram showing a volume data having exemplary rendering directions.
- FIG. 5: is a schematic diagram showing an example of display region on a screen of a display unit.
- FIGS. 6 to 8: are schematic diagrams showing storage areas allocated in a second memory disposed within the memory.
- FIG. 9: is a schematic diagram showing storage areas allocated in a third memory provided within the memory.
- FIGS. 10 to 12: are schematic diagrams showing examples of displaying three-dimensional ultrasound images according to display setting information.

### DETAILED DESCRIPTION OF THE INVENTION

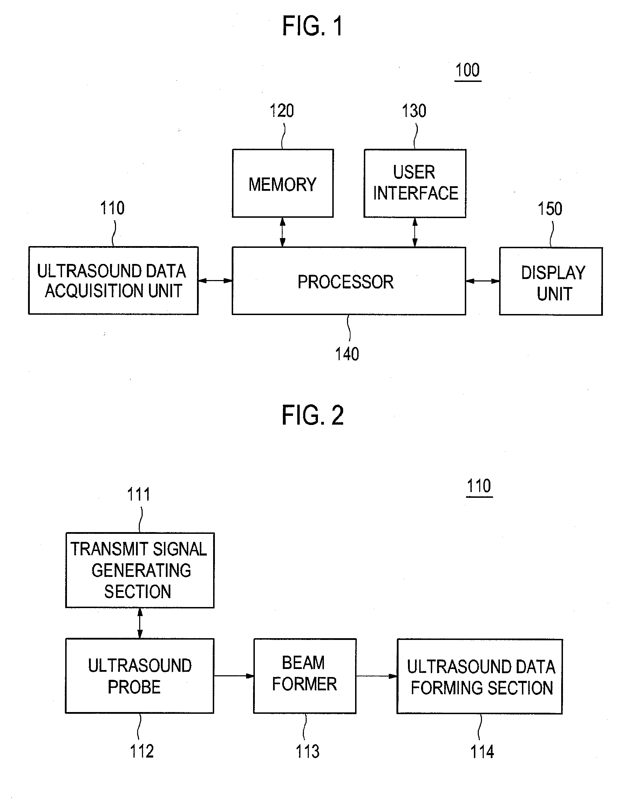

FIG. 1 is a block diagram showing an illustrative embodiment of an ultrasound system. The ultrasound system 100 may include an ultrasound data acquisition unit 110, a memory 120, a user interface 130, a processor 140 and a display unit 150.

The ultrasound data acquisition unit 110 may be configured to transmit ultrasound signals to a target object (not shown) and receive ultrasound echo signals reflected from the target object. The ultrasound data acquisition unit 110 may be further configured to acquire ultrasound data based on the received ultrasound echo signals.

FIG. 2 is a block diagram showing the ultrasound data acquisition unit 110 provided within the ultrasound system, which id shown in FIG. 1. The ultrasound data acquisition unit 110 may include a transmit (Tx) signal generating section 111, an ultrasound probe 112 including a plurality of transducer elements (not shown), a beam former 113 and an ultrasound data forming section 114.

The Tx signal generating section 111 may generate Tx signals according to an image mode set in the ultrasound system 100. The image mode may include a brightness (B) mode, a Doppler (D) mode, a color flow mode, etc. In one exemplary embodiment, the B mode is set in the ultrasound system 100 to obtain a B-mode image.

The ultrasound probe 112 may receive the Tx signals from the Tx signal generating section 111 and generate ultrasound signals, which may travel into the target object. The ultrasound probe 112 may further receive ultrasound echo signals reflected from the target object, and convert them into electrical receive signals. In such a case, the electrical receive signals may be analog signals, which may form a plurality of image frames by repeatedly performing the transmission and reception of the ultrasound signals. The ultrasound probe 112 may be a three-dimensional probe, a two-dimensional probe, a one-dimensional probe or the like.

The beam former 113 may convert the electrical receive signals outputted from the ultrasound probe 112 into digital signals. The beam former 113 may further apply delays to the digital signals in consideration of the distances between the transducer elements and focal points to thereby output receive-focused beams.

The ultrasound data forming section 114 may form a plurality of ultrasound data corresponding to the plurality of image frames by using the receive-focused beams. The plurality of ultrasound data may be radio frequency (RF) data or IQ data.

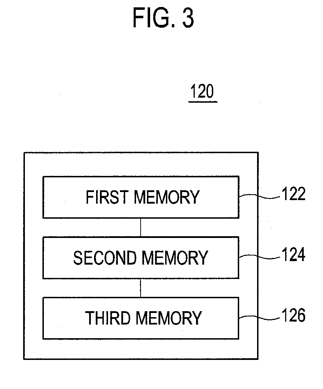

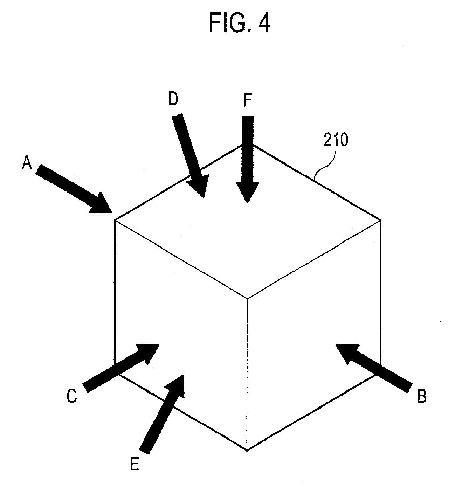

FIG. 3 is a schematic diagram showing the memory 120 located within the ultrasound system, which is shown in FIG. 1. The memory 120 may include a first memory 122, a second memory 124 and a third memory 126. The first memory 122 may store a plurality of rendering setting information, region of interest (ROI) setting information, display setting information, mode setting information and the at least one three-dimensional ultrasound image. The rendering setting information may contain information on at least two rendering directions. For example, as shown in FIG. 4, the rendering setting information may include the information on six rendering directions A to F on a volume data 210 for forming six three-dimensional ultrasound images. However, it is noted herein that the rendering directions should not be limited thereto. The ROI setting information may include information on a size of ROI and a position of the ROI set on at least one of the three-dimensional ultrasound images. The display setting information may include information for selecting at least one three-dimensional ultrasound image for displaying among a plurality of three-dimensional ultrasound images and for selecting at least one sub-display region of the display region for displaying at least one selected three-dimensional ultrasound image.

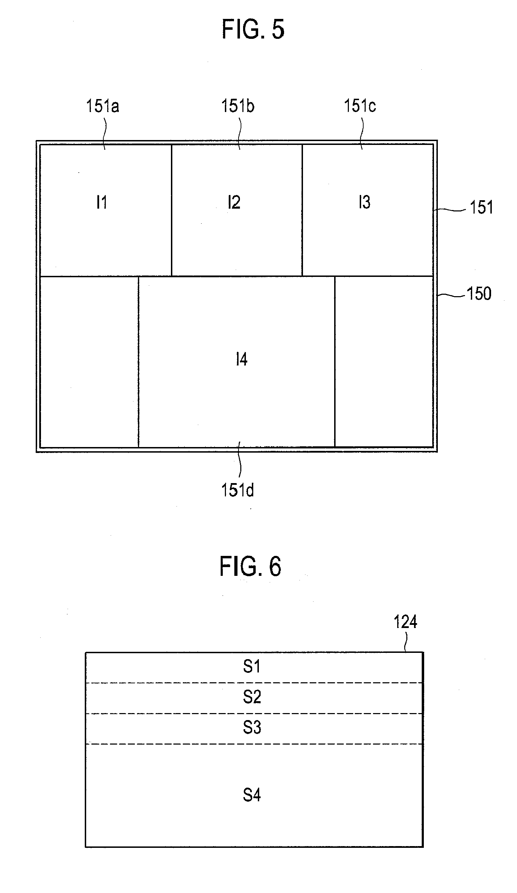

FIG. 5 is a schematic diagram showing an example of display regions on a screen of a display unit 150, which is shown in FIG. 1. The display setting information stored in the first memory 122(shown in FIG. 3) may include information for selecting a plurality of sub-display regions. In FIG. 5, four three-dimensional ultrasound images are displayed on the sub-display regions 151a-151d in the display region 151 of the display unit 150.

The mode setting information stored in the first memory 122 (shown in FIG. 3) may include information for changing a display mode. The display mode may include an X-Ray mode, a Min. mode, a Max. mode, a Light mode, etc. In the X-Ray mode, the three-dimensional ultrasound image may be formed by regulating intensities of voxels using an average intensity of the voxels. The X-Ray mode is useful in displaying the three-dimensional ultrasound image such as an X-Ray image. In the Max. mode, the three-dimensional ultrasound image is formed by reconstructing the three-dimensional ultrasound image using the voxels having maximum intensities. The Max. mode is useful in displaying bones of the human body. In the Min. mode, the three-dimensional ultrasound image is formed by reconstructing the three-dimensional ultrasound image using the voxels having minimum intensities. The Min. mode is useful in displaying vessels and hollows of the human body. In the Light mode, the three-dimensional ultrasound image is formed by transforming information on depth of each voxel into information on brightness.

Referring now back to FIG. 3, the second memory 124 may store at least one three-dimensional ultrasound image selected through the user interface 130. Referring to FIGS. 5 and 6, when three-dimensional ultrasound images I1-I4 are displayed on first to fourth sub-display regions 151a-151d, first to fourth storage areas S1-S4 corresponding to the first to fourth sub-display regions 151a-151d may be allocated in the second memory 124. The three-dimensional ultrasound images I1-I4 may be stored in the first to fourth storage areas S1-S4. The first to fourth storage areas S1-S4 may include information on positions of the respective first to fourth sub-display regions 151a-151d.

If a storing capacity of the second memory 124 is smaller than the storing capacity of the third memory 126, which may function as a image frame buffer memory, then the selected three-dimensional ultrasound images may be transferred from the first memory 122 to the second memory 124 one by one. Thereafter, they may be transferred to the third memory 126, where all the transferred three-dimensional ultrasound images may form image frame data, which will be transferred to the display unit 150.

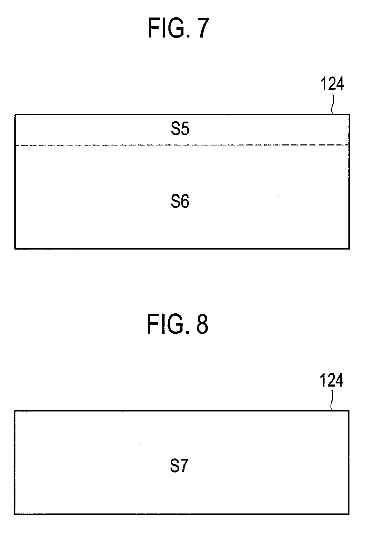

Referring to FIG. 7, a fifth storage area S5 corresponding to one of the first to third sub display regions 151a-151c and sixth storage area S6 corresponding to the fourth display region 151d may be allocated in the second memory 124. For example, the processor 140 may store the three-dimensional ultrasound image I1 in the fifth storage area S5, and then transfer the three-dimensional ultrasound image I1 from the second memory 124 to the third memory 126. The processor may then fetch the three-dimensional ultrasound image I2 from the first memory 122 and store the three-dimensional ultrasound image I2 in storage area S5. It maythen transfer the three-dimensional ultrasound image I2 from the second memory 124 to the third memory 126. Thereafter the processor 140 may fetch the three-dimensional ultrasound image I3 and store the three-dimensional ultrasound image I3 in storage area S5. It may thentransfer the three-dimensional ultrasound image I3 from the second memory 124 to the third memory 126. The processor 140 may then fetch the three-dimensional ultrasound image I4 from the first memory 122 and store the three-dimensional ultrasound image I4 in the sixth storage area S6, and then transfer the three-dimensional ultrasound image I4 from the second memory 124 to the third memory 126.

Referring to FIG. 8, a seventh storage area S7 corresponding to the fourth display region 151d may be allocated in the second memory 124. For example, the processor 140 may fetch the three-dimensional ultrasound images I1-I4 from the first memory 122 one by one in this order and store the three-dimensional ultrasound images I1-I4 in the seventh storage area S7, respectively. It may then transfer the three-dimensional ultrasound image I1-I4 one by one from the second memory 124 to the third memory 126 where the image frame data may be formed.

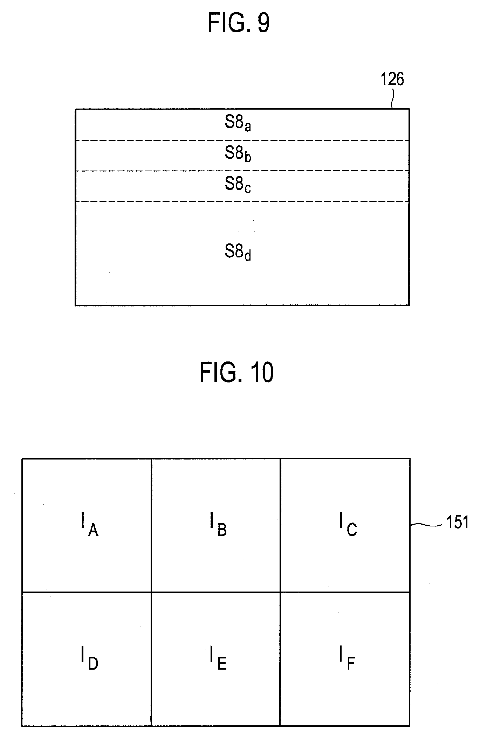

The third memory 126 may store the at least one three-dimensional ultrasound image to be displayed on the display region. The third memory 126 may store the three-dimensional ultrasound images, which are previously stored in the second memory 124. The three-dimensional ultrasound images stored in the third memory 126 may be displayed on the display unit 150. Referring to FIGS. 5 and 9, the third memory 126 may include sub-storage areas S8ₐ-S8_{d} corresponding to the first to fourth sub-display regions 151a-151d. The first to fourth sub-storage areas S8ₐ-S8_{d} correspond to the respective first to fourth sub-display regions 151a-151d. The three-dimensional ultrasound image I1 may be stored in the first sub-storage area S8ₐ in the third memory 126 and displayed on the first sub-display region 151a. The three-dimensional ultrasound image I2 may be stored in the second sub-storage area S8_{b} in the third memory 126 and displayed on the second sub-display region 151b. The three-dimensional ultrasound image I3 may be stored in the third sub-storage area S8_{c} in the third memory 126 and displayed on the third sub-display region 151c. The three-dimensional ultrasound image I4 is stored in the fourth sub-storage area S8_{d} in the third memory 126 and displayed on the fourth sub-display region 151d.

Referring back to FIG. 1, the user interface 130 may include a control panel (not shown), a mouse (not shown) and a keyboard (not shown). The user interface 130 may allow a user to input user instructions. The user instructions may include first and second user instructions. The first user instruction may include at least one of the rendering setting instruction, the ROI setting instruction, the display setting instruction and the mode setting instruction. The first user instruction may be stored in the first memory 122. The second instruction may include at least one of a rendering setting information selecting instruction, a ROI setting information selecting instruction, a display setting information selecting instruction and a mode setting information selecting instruction. The rendering setting information selecting instruction is an instruction for selecting one of the rendering setting information stored in the first memory 122. The ROI setting information selecting instruction is an instruction for selecting one of the ROI setting information stored in the first memory 122. The display setting information selecting instruction is an instruction for selecting one of the display setting information stored in the first memory 122. The mode setting information selecting instruction is an instruction for selecting one of the mode setting information stored in the first memory 122.

The processor 140 may form the volume data including a plurality of voxels based on the plurality of ultrasound data. The processor 140 may be embodied as a central processing unit (CPU) or a graphic processing unit (GPU). When the rendering setting information is inputted through the user interface 130, the processor 140 may render the volume data along the rendering directions set according to the rendering setting information to thereby form at least one three-dimensional ultrasound image. The processor 140 may be further operable to allocate at least one storage area corresponding to the three-dimensional ultrasound images in the second memory 124, as illustrated in FIGS. 6 to 8. The formed three-dimensional ultrasound images may be stored in the storage areas in the second memory 124. The processor 140 may allocate at least one storage area in the third memory 126 according to the selected rendering setting information. The three-dimensional ultrasound images, which are stored in the corresponding storage area in the second memory 124, may be stored in the corresponding storage areas in the third memory 126. When the storage areas of the third memory 126 are filled with the three-dimensional ultrasound images, the processor 140 may divide the display region 151 of the display unit 150 into a plurality of sub-display regions. The three-dimensional ultrasound images stored in the storage areas of the third memory 126 may be displayed on the display region 151 of the display unit 150. When the rendering setting information selecting instruction is inputted through the user interface 130, the processor 140 may load the rendering setting information from the first memory 122 corresponding to the rendering setting information selecting instruction. The processor 140 may render the volume data along the rendering directions corresponding to the loaded rendering setting information from the first memory 122 to form three-dimensional ultrasound images. The processor 140 may divide the second memory 124 into at least one sub-storage area as illustrated in FIGS. 6 to 8. The formed three-dimensional ultrasound images may be stored in the corresponding storage areas of the second memory 124. The processor 140 may divide the third memory 126 into the sub-storage areas according to the rendering setting information. The three-dimensional ultrasound images, which are stored in the storage areas of the second memory 124, may be stored in the storage areas of the third memory 126. When the storage areas of the third memory 126 are filled with the three-dimensional ultrasound images, the processor 140 may divide the display region 151 of the display unit 150 into a plurality of sub-display regions. The three-dimensional ultrasound images stored in the storage areas of the third memory 126 may be displayed on the display region 151 of the display unit 150. The processor 140 may render the volume data along at least two directions.

When the ROI setting information is inputted through the user interface 130, the processor 140 may be operable to set a ROI on the volume data corresponding to the ROI setting information. The processor 140 may extract volume data corresponding to the ROI. The processor 140 may form the three-dimensional ultrasound image corresponding to the ROI by rendering the extracted volume data. The formed three-dimensional ultrasound image may be stored in the second memory 124. When the ROI setting information selecting instruction is inputted through the user interface 130, the processor 140 may load the ROI setting information from the first memory 122 corresponding to the ROI setting information selecting instruction. The processor may set the ROI on the volume data and extract volume data corresponding to the ROI by using the loaded ROI setting information. The processor 140 may render the extracted volume data to form the three-dimensional ultrasound image corresponding to the ROI. The processor 140 may also form two-dimensional ultrasound image corresponding to the ROI.

When the display setting information is inputted through the user interface 130, the processor 140 may set the display region 151 of the display unit 150, allocate the storage areas in the second and third memories 124, 126 and display the three-dimensional ultrasound images stored in the third memory 126 according to the display setting information.

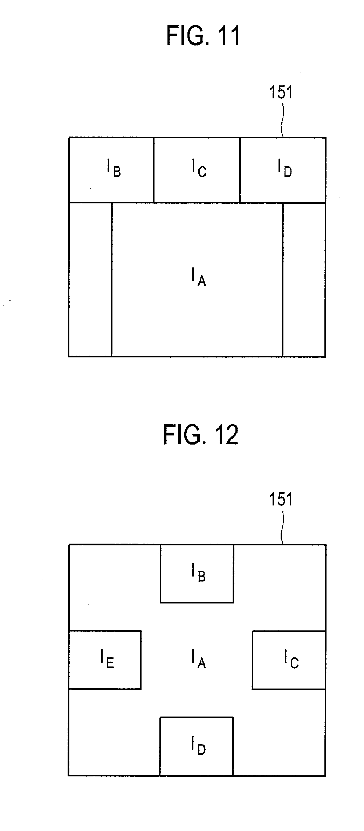

FIGS. 10 to 12 are schematic diagrams showing examples of displaying three-dimensional ultrasound images according to display setting information. For example, when the display setting information, including selection information for selecting four three-dimensional ultrasound images I_{A}-I_{D} among sixth three-dimensional ultrasound images I_{A}-I_{F} and position information for displaying the four three-dimensional ultrasound images I_{A-}I_{D} on the display region 151 of the display unit 150 as shown in FIG. 11, is inputted through the user interface 130, the processor 140 may allocate the storage area in the second memory 124 (shown in FIGS. 6 to 8) according to the display setting information and store the four three-dimensional ultrasound images I_{A}-I_{D} in each storage area. The processor 140 may be operable to allocate the storage areas in the third memory 126 according to the display setting information. The four three-dimensional ultrasound images I_{A}-I_{D}, which are stored in the corresponding storage area of the second memory 124, may be transferred to the storage areas in the third memory 126. When the storage areas of the third memory 126 are filled with the four three-dimensional ultrasound images I_{A}-I_{D}, the processor 140 may divide the display region 151 of the display unit 150 according to the display setting information and transfer the image frame data from the third memory 126 to the display unit 150. Thereafter, the four three-dimensional ultrasound images I_{A}-I_{D} stored in the third memory 126 may be displayed on the display region 151 of the display unit 150.

As another example, when the display setting information, including selection information for selecting five three-dimensional ultrasound images I_{A}-I_{E} among sixth three-dimensional ultrasound images I_{A}-I_{F} and position information for displaying the five three-dimensional ultrasound images I_{A}-I_{E} on the display region 151 of the display unit 150 as shown in FIG. 12, is inputted through the user interface 130, the processor 140 may allocate the storage area in the second memory 124 according to the display setting information and store the five three-dimensional ultrasound images I_{A}-I_{E} in each storage area. The processor 140 may allocate the storage areas in the third memory 126 according to the display setting information. The five three-dimensional ultrasound images I_{A}-I_{E}, which are stored in the corresponding storage area of the second memory 124, may be stored in the storage areas of the third memory 126. When the storage areas of the third memory 126 are filled with the five three-dimensional ultrasound images I_{A}-I_{E}, the processor 140 may divide the display region 151 of the display unit 150 according to the display setting information and transfer the image frame data from the third memory 126 to the display unit,. For example, and display the five three-dimensional ultrasound images I_{A}-I_{E} stored in the third memory 126 on the display region 151 of the display unit 150.

When the mode setting information is inputted through the user interface 130, the processor 140 may be operable to perform data processing according to a display mode selected in response to the inputted mode setting information. When the mode setting information selecting instruction is inputted through the user interface 130, the processor 140 may load the mode setting information from the first memory 122 corresponding to the inputted mode setting information selecting instruction. The processor 140 may operate data processing according to the selected display mode in response to the loaded mode setting information.

The display unit 150 may display the three-dimensional ultrasound images formed at the processor 140. The display unit 150 may include the display region 151 for displaying the three-dimensional ultrasound images as shown in FIGS. 10 to 12.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a ultrasound data acquisition unit configured to transmit ultrasound signals to a target object, receive ultrasound echo signals reflected from the target object and acquire ultrasound data based on the received ultrasound echo signals;
a user interface configured to allow a user to input rendering setting information containing information on at least two rendering directions;
a processor in communication with the ultrasound data acquisition unit and the user interface, the processor being configured to form volume data by using the ultrasound data, render the volume data along the at least two rendering directions and form three-dimensional ultrasound images corresponding to the at least two rendering directions;
a memory in communication with the processor and being configured to store the three-dimensional ultrasound images; and
a display unit in communication with the processor and being configured to display the three-dimensional ultrasound images on a display region thereof.

2. The ultrasound system of Claim 1, wherein the user interface is further configured to receive region of interest (ROI) setting information for setting at least one ROI on the three-dimensional ultrasound images; and
wherein the processor is further configured to set the at least one ROI on the volume data according to the ROI setting information, extract volume data corresponding to the at least one ROI and render the extracted volume data to form at least one three-dimensional ultrasound image corresponding to the at least one ROI.

3. The ultrasound system of Claim 2, wherein the user interface is further configured to receive display setting information for displaying the at least one three-dimensional ultrasound image on at least one sub-display region; and
wherein the processor is further configured to divide the display region according to the display setting information and display the at least one three-dimensional ultrasound image on the display region.

4. The ultrasound system of Claim 3, wherein the user interface is further configured to receive mode setting information for changing a display mode of the at least one three-dimensional ultrasound image; and
wherein the processor is further configured to operate data processing for changing the display mode of the at least one three-dimensional ultrasound image.

5. The ultrasound system of Claim 4, wherein the display mode includes an X-Ray mode for forming the three-dimensional ultrasound images by regulating intensities of voxels using an average intensity of the voxels, a Min. mode for forming the three-dimensional ultrasound images by reconstructing the three-dimensional ultrasound images using the voxels having minimum intensities, a Max. mode for forming the three-dimensional ultrasound images by reconstructing the three-dimensional ultrasound images using the voxels having maximum intensities, and a Light mode for forming the three-dimensional ultrasound images by transforming information on depth of the voxels into information on intensity of the voxels.

6. The ultrasound system of Claim 5, wherein the memory comprises:
a first memory configured to store the rendering setting information, the ROI setting information, the display setting information, the mode setting information and the at least one three-dimensional ultrasound image;
a second memory configured to store the at least one three-dimensional ultrasound image selected through the user interface; and
a third memory configured to store the at least one three-dimensional ultrasound image to be displayed on the display region.

7. A method of providing three-dimensional ultrasound images in an ultrasound system comprising:
a) obtaining ultrasound data by the ultrasound data acquisition unit within the ultrasound system;
b) receiving rendering setting information containing information on at least two rendering directions by a user interface within the ultrasound system;
c) forming a volume data by using the ultrasound data by a processor within the ultrasound system;
d) rendering the volume data along the at least two directions according to the rendering setting information by the processor;
e) forming three-dimensional ultrasound images corresponding to the at least two directions by the processor; and
f) displaying the three-dimensional ultrasound images on a display region by the display unit within the ultrasound system.

8. The method of Claim 7, wherein the step a) comprises:
a1) transmitting ultrasound signals to a target object by the ultrasound data acquisition unit within the ultrasound system;
a2) receiving ultrasound echo signals reflected from the target object by the ultrasound data acquisition unit; and
a3) obtaining ultrasound data based on the received ultrasound echo signals by the ultrasound data acquisition unit.

9. The method of Claim 7 further comprising:
g) receiving region of interest (ROI) setting information for setting ROI on the at least one three-dimensional ultrasound image by the user interface;
h) setting the ROI on the volume data according to the ROI setting information by the processor;
i) extracting volume data corresponding to the ROI by the processor; and
j) forming three-dimensional ultrasound images corresponding to the ROI by rendering the extracted volume data by the processor.

10. The method of Claim 7 further comprising:
g) receiving display setting information for displaying at least one three-dimensional ultrasound images on at least one sub-display region by the user interface;
h) dividing the display region into the at least one sub-display region according to the display setting information by the processor; and
i) displaying the at least one three-dimensional ultrasound images on the at least one sub-display region by the processor.

11. The method of Claim 7 further comprising:
g) receiving mode setting information for changing display mode of at least one three-dimensional ultrasound image by the user interface; and
h) operating data processing for changing the display mode of the at least one three-dimensional ultrasound image
by the processor.

12. A computer readable medium comprising instructions that, when executed by a processor, perform a method of providing three-dimensional ultrasound images, comprising steps of:
a) obtaining ultrasound data;
b) receiving rendering setting information containing information on at least two rendering directions;
c) forming a volume data by using the ultrasound data;
d) rendering the volume data along the at least two directions according to the rendering setting information;
e) forming three-dimensional ultrasound images corresponding to the at least two directions; and
f) displaying the three-dimensional ultrasound images.

13. The computer readable medium of Claim 12, wherein the steps further comprises:
g) receiving region of interest (ROI) setting information for setting ROI on the at least one three-dimensional ultrasound images;
h) setting the ROI on the volume data according to the ROI setting information;
i) extracting volume data corresponding to the ROI; and
j) forming three-dimensional ultrasound images corresponding to the ROI by rendering the extracted volume data.

14. The computer readable medium of Claim 12, wherein the steps further comprises:
g) receiving display setting information for displaying at least one three-dimensional ultrasound image on at least one sub-display region;
h) dividing the display region into the at least one sub-display region according to the display setting information; and
i) displaying the at least one three-dimensional ultrasound image on the at least one sub-display region.

15. The computer readable medium of Claim 12, wherein the steps further comprises:
g) receiving mode setting information for changing display mode of at least one three-dimensional ultrasound image; and
h) changing the display mode of the at least one three-dimensional ultrasound image.
